# FASCICULE DE BREVET EUROPEEN

(11) **EP 1 581 177 B1**
(45) Date de publication et mention de la délivrance du brevet: **11.04.2018**
(21) Numéro de dépôt: 03814486.1
(22) Date de dépôt: 23.12.2003
(51) Int. Cl.: A61Q 19/08, A61K 8/60, A61K 8/64, A61K 8/66, A61K 31/7076, A61K 38/06, A61K 38/08, A61K 38/34, A61K 38/43, A61K 38/57, A61Q 1/14, A61Q 17/04, A61Q 19/00, A61Q 19/04, A61Q 19/06

(54) **BIO-ACTIVATEUR METABOLIQUE CUTANE**
HAUT-STOFFWECHSELBIOAKTIVATOR
CUTANEOUS METABOLIC BIO-ACTIVATOR

(30) Priorité: 30.12.2002 FR 0216871; 30.12.2002 FR 0216872; 30.12.2002 FR 0216873; 30.12.2002 FR 0216874
(43) Date de publication de la demande: 05.10.2005
(73) Titulaire: Thorel, Jean-Noël, 75014 Paris (FR)
(72) Inventeur: THOREL, Jean-Noel, F-75014 Paris (FR); REDZINIAK, Gérard, F-92160 Antony (FR)
(74) Mandataire: Cabinet Laurent & Charras
(86) Numéro de dépôt international: PCT/FR2003/003883
(87) Numéro de publication internationale: WO 2004/060393

(56) Documents cités:
- EP-A- 0 571 198
- EP-A- 0 707 844
- WO-A-02/085927
- DD-A- 268 157
- DE-A- 4 139 639
- DE-A- 10 032 964
- FR-A- 2 546 164
- FR-A- 2 668 365
- FR-A- 2 706 300
- FR-A- 2 783 169
- US-A- 4 844 884
- US-A- 5 254 331
- US-A- 5 885 974
- US-A- 5 958 976
- ANONYMOUS: "Skin energy" INTERNET ARTICLE, [Online] XP002286181 Cognis Extrait de l'Internet: URL:http://www.cognis.com/carechemicals/Be auty/beauty_popup_gen1_SkinEnergy.html> [extrait le 2004-06-08]
- MATSURA I ET AL: "Skin roughness-treating cosmetics containing carnosines and mucopolysaccharides, peptide and/or lecithin" 24 août 1992 (1992-08-24), CHEMICAL ABSTRACTS + INDEXES, AMERICAN CHEMICAL SOCIETY. COLUMBUS, US, PAGE(S) 436 , XP002966006 ISSN: 0009-2258 abrégé
- PATENT ABSTRACTS OF JAPAN vol. 005, no. 194 (C-082), 10 décembre 1981 (1981-12-10) & JP 56 115707 A (MITSUBISHI CHEM IND LTD), 11 septembre 1981 (1981-09-11)
- DATABASE WPI Derwent Publications Ltd., London, GB; AN 2003-451671 XP002251028 NN: "Superoxide dismutase-like substances useful in skin cosmetics for prepventing aging and wrinkles of skin adn improving fairness of skin, comprises extract of Ehretia belonging to Boraginaceae, as active ingredient" & JP 2002 363087 A (MARUZEN SEIYAKU KK) 18 décembre 2002 (2002-12-18)
- RC PEPE, ET AL. (EDS.): "International Cosmetic Ingredient Dictionary and Handbook"", 2002, CTFA, WASHINGTON DC * page 36 *
- NN: 'Biophos 35 Glucophosphoprotein', [en ligne] 2006, pages 1 - 3 ARCH (TM) COSMETIC INGREDIENTS & IDEAS Extrait de l'Internet: <URL:http://www.archchemicals.com/Fed/PC/Pr oducts/Biotechnological/Biophos35.htm> [extrait le 2007-09-14]
- DATABASE GNPD [en ligne] MINTEL 01 Décembre 2001 INTER-FRAGRANCES: 'Oligo Aqua Vita Moisturizing Day Cream' Database accession no. 124877
- DATABASE GNPD [en ligne] MINTEL INTER-FRAGRANCES: 'Oligo Aqua Vita Eye Shadow Lightening Cream' Database accession no. 124933

## Description

La présente demande concerne de manière générale les compositions cosmétiques.

La présente demande a pour objet de mettre en oeuvre par la voie cosmétique, un nouveau concept de viabilité et/ou stimulation cellulaire cutanée, désigné sous le vocable de bioactivité métabolique. Plus particulièrement, l'invention concerne un bioactivateur métabolique cutané.

Le mode de vie de l'individu, l'environnement agressif et l'évolution chrono-biologique dégénérative ont pour conséquence que les fonctions biologiques et facultés vitales des tissus cutanés s'affaiblissent dans le temps. Il apparaît donc essentiel de rétablir ou maintenir un bon fonctionnement métabolique et catabolique des cellules de la peau (kératinocytes, cellules de Langerhans, mélanocytes, fibroblastes, etc...) pour que celles-ci échangent avec leur environnement, et de l'énergie exogène, et de l'information fonctionnelle.

Le document DE 4139639 concerne des extraits aqueux synthétiques d'organes utilisés dans la préparation de compositions cosmétiques et pour activation du métabolisme cellulaire. Ces extraits comprennent, entre autres, un dérivé d'acide nucléique tel que de l'ATP-Mg. Ces extraits comprennent aussi au moins un acide aminé et un dérivé peptidique définis et considéré comme essentiels pour l'activité de l'extrait synthétique d'organe. Il est indiqué que d'autres aminoacides peuvent être présents dans la composition dont la L-carnosine, sans cependant lier la présence de ces aminoacides optionnels à un quelconque effet.

Le document FR 2 668 365 divulgue que la carnosine possède une activité anti-oxydante, cicatrisante et anti-inflammatoire et qu'elle peut être associée à un acide gras pour améliorer sa pénétration cutanée.

Le document Matsura et al., (1992, Chemical abstracts, American Chemical Society, Colombus, US, Vol. 117, page 436) divulgue des compositions cosmétiques pour traiter la rugosité de la peau comprenant des carnosines, des mucopolysaccarides, des peptides et ou de la lécithine.

La présente demande a donc pour objet d'augmenter ou corriger, naturellement, les capacités vitales de la peau, par la conjonction d'un apport exogène d'énergie et de la stimulation de messages intercellulaires. La synergie entre cet apport et cette stimulation permet à la peau de réagir contre toute agression ou tout dysfonctionnement, et ce en activant des mécanismes métaboliques préexistant dans la peau (moléculaires, cellulaires, tissulaires), et en optimisant le cas échéant l'interaction entre la peau et le ou les actif(s) cosmétique(s) apporté(s) par des soins ou traitements dermo-cutanés.

A cette fin, la présente invention propose une composition cosmétique comprenant un système bioactif associant, d'une part une forme stable en solution aqueuse d'ATP (adenosine-triphosphate) sous la forme d'un sel de sodium d'ATP, avec éventuellement du Gp4G (diguanosine tetraphosphate) ou de l'Ap4A (diadénosine tetraphosphate), et d'autre part au moins un dipeptide biomimétique, mimant un polypeptide cutané ou une protéine cutanée, ou une biomolécule agoniste ou antagoniste audit peptide ou à ladite protéine, ledit dipeptide biomimétique étant la β-alanyl-L-hystidine.

Par « composition cosmétique », on entend toute composition ayant pour fonction de maintenir, restaurer, ou améliorer l'aspect des parties superficielles du corps humain, à titre principal de la peau, et ce quelque soit le mode d'administration de ladite composition, à savoir par voie externe topique, ou par voie interne orale.

Par « précurseur d'ATP », on entend le Gp₄G (ou diguanosine tetraphosphate) ou l'Ap₄A (diadénosine tetraphosphate).

Par « dipeptide biomimétique », on entend un peptide constitué de deux acides aminés, mimant un peptide cutané ou une protéine cutanée, ou une biomolécule agoniste ou antagoniste audit peptide ou à ladite protéine, lequel ou laquelle joue un rôle ou intervient dans une biosynthèse cutanée ou le transfert d'une information cutanée.

Par « mimer » ou « mimétisme », on entend la caractéristique selon laquelle le peptide considéré exerce in vitro, et en particulier dans une composition selon l'invention, un effet biologique similaire à ou proche d'une fonction biologique in vivo (par exemple dans la peau) d'une biomolécule de référence (par exemple peptide ou protéine).

Par « peptide », dans toute la description et les revendications, il faut entendre aussi bien une suite de plusieurs acides aminés non substitués, qu'une suite des mêmes acides aminés, dont certains, par exemple l'acide aminé N-terminal et / ou l'acide aminé C-terminal sont substitués par un groupement ou substituant, fonctionnel ou non.

Ces peptides peuvent être obtenus soit par synthèse chimique classique (en phase solide ou en phase homogène liquide), soit par synthèse enzymatique (Kullman et al., J. Biol. Chem. 1980, 255, 8234) à partir des acides aminés constitutifs ou de leurs dérivés.

Ces peptides peuvent être obtenus également par fermentation d'une souche de bactérie modifiée ou non par génie génétique, pour produire les séquences recherchées ou leurs différents fragments.

Enfin, ces peptides peuvent être obtenus par extraction de protéines d'origine animale ou végétale, préférentiellement végétale, suivie d'une hydrolyse contrôlée qui libère les peptides en question. De nombreuses protéines trouvées dans les plantes sont susceptibles de contenir des séquences intéressantes au sein de leur structure. L'hydrolyse ménagée permet de dégager ces fragments peptidiques.

Selon l'invention, le système bioactif retenu au sein de la composition cosmétique potentialise un ou plusieurs principes actifs, présents au sein de celle-ci.

Grâce à l'invention, le système bioactif caractérisant cette dernière permet tout à la fois de restaurer et / ou de maintenir l'activité naturelle de l'épiderme. Ceci est particulièrement vérifié si la composition cosmétique contient de plus des nutriments de la peau, et / ou une phase aqueuse assurant la viabilité des cellules cutanées.

La présente invention présente encore les modes d'exécution suivants :
- la forme stable d'ATP est un sel de sodium, par exemple un sel disodique d'ATP,
   le peptide biomimétique est l'histidine-β-alanyl (mime la superoxyde-dismutase),
- le peptide biomimétique est un dipeptide ;

La quantité efficace d'actif(s) éventuellement présent(s) dans une composition selon l'invention correspond à la quantité nécessaire pour obtenir le résultat désiré, et les compositions suivant l'invention dépendent de l'usage auxquels ces compositions sont destinées.

Une première catégorie comprend les compositions cosmétiques destinées à être appliquées sur une peau saine, afin d'en améliorer l'esthétique et le confort. Une peau saine peut être définie comme exempte de pathologie, mais sans pour autant être en parfait état. Cette peau peut présenter des signes de sécheresse, des signes d'irritation, des rides liées au vieillissement chronologique ou actinique, des zones d'hyper-sécrétion de sébum, d'hyper ou d'hypo-pigmentation. De plus, la peau saine peut avoir besoin d'une photoprotection temporaire ou permanente, afin de mieux résister à la lumière solaire.

Une autre catégorie comprend les compositions destinées à être appliquées sur des peaux fragilisées par la maladie ou des médications, soit à titre préventif, soit au titre d'un traitement relais à des traitements médicaux.

Les compositions cosmétiques selon la présente invention peuvent encore comprendre au moins un actif cosmétique choisi parmi les antioxydants, les agents anti-radicaux libres, les α-hydroxyacides, les vitamines, les filtres ou écrans solaires, les agents répulsifs contre les insectes, les anti-inflammatoires.

Bien entendu, l'homme de l'art veillera à choisir ce ou ces éventuels actifs, et/ou leurs quantités, de manière telle que les propriétés avantageuses du système bioactif selon l'invention ne soient pas, ou substantiellement pas, altérées par la ou les adjonctions envisagées. De préférence une synergie entre le système bioactif selon l'invention, et le ou les actifs considérés, sera recherchée.

Les compositions de l'invention peuvent être préparées selon les techniques bien connues de l'homme de l'art, en particulier celles destinées à la préparation d'émulsions de type huile-dans-eau (HIE) ou eau-dans-huile (E/H).

Ces compositions peuvent se présenter en particulier sous forme d'émulsion, simple ou complexe : double (H/E ou E/H) ou triple (E/H/E ou H/E/H), telle qu'une crème, un lait, un gel ou un gel-crème ; de poudre, de bâtonnet solide, et éventuellement être conditionnées en aérosol, et se présenter sous forme de mousse ou de spray.

Lorsque la composition cosmétique selon l'invention est utilisée pour la protection ou le soin de l'épiderme humain, ou comme composition antisolaire, elle peut se présenter sous forme de suspension ou de dispersion dans des solvants ou des corps gras, sous forme de dispersion vésiculaire non ionique, ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, et constituer par exemple une composition à rincer, à appliquer avant ou après shampooing, avant ou après coloration ou décoloration, avant, pendant ou après permanente ou défrisage ; cette composition peut aussi se présenter sous forme d'une lotion ou d'un gel coiffant ou traitant, d'une lotion ou d'un gel pour le « brushing » ou la mise en plis, d'une composition de permanente ou de défrisage, de coloration ou décoloration des cheveux.

Lorsque la composition est utilisée comme produit de maquillage des ongles, des cils, des sourcils ou de la peau, tel que crème de traitement de l'épiderme, fond de teint, bâton de rouge à lèvres, fard à paupières, fard à joues, mascara ou ligneur, encore appelé « eye liner », elle peut se présenter sous forme solide ou pâteuse, anhydre ou aqueuse, comme des émulsions huile-dans-eau ou eau-dans-huile, des dispersions vésiculaires non ioniques ou encore des suspensions.

Pour les compositions selon l'invention, le pH sera physiologique, compris entre 4 et 7. Lorsqu'elle est appliquée par voie topique, la composition, comprenant au moins un ATP et éventuellement un précurseur, conjugués à au moins un peptide biomimétique, peut être appliquée sur le visage, le cou, le cuir chevelu, les muqueuses, les ongles, le corps, la poitrine, les pieds, les jambes, ou toute autre partie du corps.

Les compositions de l'invention peuvent comprendre en outre des adjuvants cosmétiques classiques, notamment choisis parmi les corps gras, les solvants organiques autres que ceux utilisés spécifiquement dans le cadre de la présente invention, les émulsionnants, les épaississants ioniques ou non ioniques, les adoucissants, les opacifiants, les stabilisants, les émollients, les silicones, les agents anti-mousse, les agents hydratants, les vitamines, les parfums, les conservateurs, les tensioactifs, les charges, les polymères, les propulseurs, les agents alcalinisants ou acidifiants, les colorants ou tous autres ingrédients habituellement utilisés en cosmétique, en particulier pour la fabrication de compositions sous forme d'émulsions. A titre d'exemple, les compositions selon l'invention comprennent des agents filtrants ou réflecteurs, dans le cas de produits destinés à être utilisés en environnement externe et sous le soleil.

Les corps gras peuvent être constitués par une huile ou une cire, ou leurs mélanges, et ils comprennent également les acides gras, les alcools gras et les esters d'acides gras. Les huiles peuvent être choisies parmi les huiles animales, végétales, minérales ou de synthèse, et notamment parmi l'huile de vaseline, l'huile de paraffine, les huiles de silicone volatiles ou non, les isoparaffines, les polyoléfines, les huiles fluorées et perfluorées. De même, les cires peuvent être choisies parmi les cires animales, fossiles, végétales, minérales ou de synthèse, connues en soi.

Parmi les huiles polaires, on peut citer l'huile connue sous la dénomination « Finsolv TN », le triméllitate de tridécyle, l'isononanoate d'isononyle, le myristate d'isopropyle, le décaprylyl carbonate, les benzoates et hydroxy-benzoates d'alcool de Guerbet, comme le produit dénommé « Hallbrite BHB » de la société CP Hall Company.

A titre indicatif, pour les formulations antisolaires conformes à l'invention, qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10 % en poids, par rapport à l'ensemble de la formulation.

De façon particulière, les compositions selon la présente invention peuvent être obtenues sous forme d'une composition anhydre qui présente des propriétés de transparence et de translucidité tout à fait remarquables.

La présente demande décrit également l'utilisation d'une composition cosmétique selon la présente invention, pour la fabrication d'un produit de soins de la peau, d'un produit de maquillage de la peau, des lèvres et/ou des phanères, anti-solaire, et d'une composition dermatologique de soin et/ou de traitement de la peau.

La composition selon l'invention peut se présenter sous la forme d'une composition teintée dermatologique, ou de soin, des matières kératiniques comme la peau, les lèvres et/ou les phanères, sous forme d'une composition de protection solaire ou d'hygiène corporelle, notamment sous forme de produit déodorant ou démaquillant, sous forme de stick. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit coloré de maquillage de la peau, en particulier un fond de teint, présentant éventuellement des propriétés de soin ou de traitement, un « blush », un fard à joues ou à paupières, un produit anti-cerne, un « eye-liner », un produit de maquillage du corps ; de maquillage des lèvres comme un rouge à lèvres, présentant éventuellement des propriétés de soin ou de traitement ; de maquillage des phanères comme les ongles, les cils, en particulier sous forme d'un mascara-pain, les sourcils et les cheveux, notamment sous forme de crayon. En particulier, la composition de l'invention peut être un produit cosmétique contenant, outre le système bioactif, des actifs cosmétiques et/ou dermatologiques.

Une composition cosmétique selon l'invention peut en outre comprendre des nacres, des pigments, ou bien encore de nanopigments (taille moyenne des particules primaires : généralement entre 5 nm et 100 nm, de préférence entre 10 nm et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium et leurs mélanges, qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Bien entendu, la composition de l'invention doit être cosmétiquement ou dermatologiquement acceptable, à savoir contenir un milieu physiologiquement acceptable, non toxique, et susceptible d'être appliquée sur la peau, les phanères ou les lèvres d'êtres humains. Par « cosmétiquement acceptable », on entend au sens de l'invention une composition d'aspect, d'odeur et de toucher agréables.

### ESSAIS

### Essai n°1 (hors invention)

On évalue l'activité bio-simulante du peptide L-citrullyl-Larginine, associé ou non à de l'ATP, sous forme de sel disodique, sur le métabolisme 20 cellulaire des kératinocytes humains normaux.

### 1. Principe

La procédure consiste à mesurer le taux d'ATP au sein d'une culture de fibroblastes en milieu appauvri en sérum (2%), en comparaison avec :
- un milieu enrichi en citrullyl-arginine, à des concentrations variables,
- un milieu enrichi en ATP, à des concentrations variables,
- un milieu enrichi en citrullyl-arginine et ATP, à des concentrations variables.

Le but de cet essai est d'évaluer l'effet activateur du mélange étudié, associé ou non à de l'ATP di-sodique, vis-à-vis de la synthèse d'ATP par des kératinocytes humains normaux.

### 2. Méthode

### ▪ Culture cellulaire

L'essai est réalisé sur une culture in vitro de kératinocytes humains normaux, ensemencés à la densité de 10⁵ cellules /puits en plaques de 6 puits.

### ▪ L-Citrullyl-L-Arginine

Afin de déterminer les concentrations applicables dans le cadre de l'essai, on réalise un test préalable de viabilité cellulaire sur kératinocytes humains normaux.

La L-Citrullyl-L-Arginine est solubilisée dans de l'eau, et la concentration finale est fixée à 0,1%.

6 dilutions d'L-Citrullyl-L-Arginine de 10⁻⁴% à 10⁻² % sont mises au contact des cellules pendant 24h et 48h. Une condition eau est réalisée comme témoin.

Pour l'essai, l'L-Citrullyl-L-Arginine sera testé aux 2 concentrations les plus fortes permettant le maintien de la viabilité cellulaire à un taux supérieur à 80% après un contact de 48h, soit 0,0001% et 0,01%.

### ▪ Irradiation UV B

Les kératinocytes, ensemencés en plaques de 6 puits à la densité de 105 cellules/puits sont cultivés en milieu standard (Epilife Sigma) pendant 48h. Avant irradiation, le milieu de culture est éliminé, les cellules sont rincées au tampon PBS et 1 ml de celui-ci est laissé au contact des cellules pendant l'irradiation. Les kératinocytes sont soumis à une irradiation UV B (312 nm) de 20 mJ/cm². Une condition non irradiée identique est réalisée en parallèle.

Après irradiation (et sur le parallèle non irradié), le PBS est éliminé et les cellules sont placées dans les différentes conditions étudiées :
- Témoin eau dans le milieu de culture
- L-Citrullyl-L-Arginine à 0,001% dans le milieu de culture
- L-Citrullyl-L-Arginine à 0,01% dans le milieu de culture

Chaque condition est réalisée en triplicate.

Après un contact de 24h ou 48h après l'irradiation, les surnageants de culture conditionnés sont récupérés. Le taux d'Endothéline-1 sécrétée par les kératinocytes est mesuré sur 100 µl de surnageant par une technique de dosage ELISA (R&D Systems, Abingdon, UK). Les niveaux d'Endotheline-1 sont calculés grâce à la courbe standard réalisée avec de l'Endothéline-1 humaine synthétisée. Les résultats sont exprimés en pg d'Endothéline-1 normés à la concentration en protéines (pg ET-1/ mg de protéines).

### 3. Résultats

L'application d'L-Citrullyl-L-Arginine pendant 24h à la concentration la plus faible testée (0,0001%) entraîne une augmentation de la sécrétion basale d'Endothéline-1 par les kératinocytes (condition non irradiée), de 20% par rapport au contrôle EAU. A la concentration supérieure (0,01%), cet effet activateur est beaucoup plus marqué : 70%.

L'irradiation UV B entraîne une stimulation de 50% de la sécrétion d'Endothéline-1 par les kératinocytes dans la condition témoin EAU. Après contact 24h avec l'L-Citrullyl-L-Arginine à 0,0001%, la sécrétion d'Endothéline-1 est augmentée de 6 % par rapport au témoin EAU irradié. A 0,001%, l'effet activateur de l'L-Citrullyl-L-Arginine est fortement majoré : 28%.

L'application d'L-Citrullyl-L-Arginine pendant 48h à la concentration la plus faible testée (0,0001%) entraîne une augmentation de la sécrétion basale d'Endothéline⁻¹ par les kératinocytes (condition non irradiée) de 26% par rapport au contrôle EAU. A la concentration supérieure : 0,01%, cet effet activateur est beaucoup plus marqué : 82%.

L'irradiation UV B entraîne une stimulation de 40% de la sécrétion d'Endothéline-1 par les kératinocytes dans la condition témoin EAU. Après contact 48h avec l'L-Citrullyl-L-Arginine à 0,0001%, la sécrétion d'Endothéline-1 est en augmentation de 4 % par rapport au témoin EAU irradié. A 0,01%, l'effet activateur de l'L-Citrullyl-L-Arginine est fortement majoré : 63%.

### 4. Conclusion

Dans les conditions expérimentales ainsi définies, pour les dilutions et les temps d'incubation choisis, il s'avère que :

La L-Citrullyl-L-Arginine, à la concentration de 0,01%, exerce un important effet activateur sur la sécrétion d'Endothéline-1 par les kératinocytes humains normaux.

En présence d'ATP 0.01% l'effet activateur est stimulé de 20%. H y a bien un effet synergique entre les molécules d'ATP et du peptide I'L-Citruilyl-L-Arginine.

Les même études réalisées avec l'ATP seul ne montrent pas d'effet sur la synthèse d'Endothéline-1.

### Essai n°2 (selon l'invention)

On étudie l'effet de l'association ATP + dipeptides sur la croissance de fibroblastes humains normaux.

Les produits étudiés sont :
- l'ATP, sous forme de sel disodique,
- la β-alanyl-I-histidine (carnosine),
- la citrullyl-arginine (exsylalgine).

### 1. Objectif de l'étude

L'objectif de cet essai est d'évaluer l'effet de l'association ATP + peptides ajoutée à un milieu de culture sur la croissance d'une lignée immortalisée de fibroblastes humains, les cellules HaCaT.

Dans cette étude, 2 peptides sont simultanément et/ou concomitamment associés à l'ATP : la β-alanyl-I-histidine (Dragoco) et la L-citrullyl-L-arginine (Exsymol)

L'étude est réalisée sur une culture effectuée dans le milieu de culture standard des cellules HaCaT, le DMEM (SIGMA) en présence de sérum de veau foetal (SVF) à 2 ou 10%.

### 2. Techniques

Les cellules HaCaT sont ensemencées à faible densité en plaque 96 puits dans le milieu standard (DMEM + 10%SVF) et poussent pendant 24h après ensemencement dans ce milieu.

Au 2éme jour, les cellules sont placées dans les différentes conditions étudiées.

Les concentrations à tester en ATP, β-alanyl-I-histidine (Carnosine) et L-citrullyl-L-arginine (exsyalgine) sont déterminées suite à des études préliminaires de cytotoxicité.

On réalise :
- Une condition témoin : milieu de culture seul + SVF
- Une condition β-alanyl-I-histidine seule à 0.5%
- Une condition L-citrullyl-L-arginine seule à 0,5%
- Une condition ATP seul à 1 µl / ml
- Une condition ATP (1 µg/ml) + β-alanyl-I-histidine (0.5%)
- Une condition ATP (1 µg/ml) + L-citrullyl-L-arginine (0,5%)
- Une condition ATP (1 µg/ml) β-alanyl-I-histidine (0.5%) + L-citrullyl-L-arginine (0,5%)

Ces différentes conditions sont réalisées à la fois dans le DMEM à 2% et à 10% SVF.

Chaque condition est réalisée en triplicate. Les milieux ne sont pas renouvelés au cours de l'expérimentation.

La densité cellulaire est évaluée 24h après l'ensemencement des cellules, avant la mise au contact des différentes conditions d'étude (= T0), puis la croissance des cellules HaCaT est évaluée au 2ème, 5ème et 7ème jours de culture par la méthode de conversion du WST-1 (lecture à 450 nm).

### 3. Résultats

La croissance cellulaire est objectivée par la mesure de la viabilité cellulaire à différents temps de l'expérimentation. Les résultats obtenus présentent le pourcentage de viabilité cellulaire calculé par rapport à la densité cellulaire initiale à T0 pour laquelle on estime avoir une densité cellulaire égale à 100%. Les effets des différents produits sur la croissance cellulaire sont analysés aux différents temps d'expérimentation par rapport au contrôle non traité au même temps d'expérimentation.
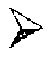 Après 2 jours de culture, on observe une absence de croissance avec maintien de la viabilité cellulaire pour la condition témoin par rapport au contrôle T0, qui s'explique par le passage en milieu appauvri en facteur de croissance (SVF 2 %).
   - L'ajout du β-alanyl-I-histidine seul à la concentration de 0.5% stimule fortement la croissance cellulaire (+22% par rapport au contrôle non traité). A la concentration de 0.1%, on n'observe aucun effet sur la croissance.
   - L'ajout d'ATP seul diminue sensiblement la viabilité cellulaire.
   - L'ajout d'ATP au β-alanyl-I-histidine entraîne une suppression de l'effet inhibiteur de l'ATP sur la viabilité cellulaire et stimule celle-ci au-delà du niveau obtenu avec le β-alanyl-I-histidine seule, et ceci pour les 2 concentrations testées.
   - L'ajout du L-citrullyl-L-arginine seuel à la concentration de 0.1 et 0.5% ne stimule pas la croissance cellulaire (+ 5%) par rapport au contrôle non traité.
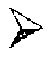 Après 5 jours de culture, on observe une légère diminution de la viabilité cellulaire par rapport au T0 due au maintien de la culture en milieu appauvri en facteur de croissance.
   - On retrouve l'effet de stimulation sur la croissance cellulaire du β-alanyl-I-histidine à la concentration de 0.5% (+12%).
   - L'ajout d'ATP seul diminue sensiblement la viabilité cellulaire, effet qui disparaît suite à l'ajout du β-alanyl-I-histidine à 0.5%.
   - La L-citrullyl-L-arginine seule ne stimule toujours pas la croissance cellulaire, quelque soit la concentration utilisée.

### 4. Conclusion

Dans les conditions expérimentales ainsi définies et aux concentrations testées, il apparaît que l'ajout d'ATP seul diminue sensiblement la viabilité de fibroblastes humains normaux après 2 à 5 jours de contact (-5 à -10%).

L'association ATP + β-alanyl-I-histidine annule l'effet inhibiteur de l'ATP sur la viabilité cellulaire et stimule celle-ci au-delà du niveau obtenu avec la β-alanyl-I-histidine seule, qui exerce un effet de stimulation de la croissance cellulaire à la concentration de 0.5%.

L'association ATP + L-citrullyl-L-arginine n'exerce aucun effet de stimulation de la croissance cellulaire, quelle que soit la concentration utilisée.

### Essai n°3 (hors invention)

### 1. Culture de cellules

Les cultures de mélanocytes humains normaux (MHN) sont réalisées à partir de prépuces d'enfants et de nouveau-nés ayant un phimosis. Les mélanocytes obtenus à partir de fragments de peau sont placés dans le milieu MCDB 153 (Sigma St Louis, MO, USA) supplémenté avec 30 µg/ml d'extrait pituitaire bovin (BPE) (Life Technologies, Paisley, Angleterre), 2% de sérum de veau foetal (SVF) (Dominique Dutscher, Brumath, France), 16 nM de phorbol-12-myristate-13-acetate (Sigma), 5µg/ml d'insuline et 1.1 µM d'hydrocortisone (Sigma). Les cultures sont maintenues dans un incubateur à 37 °C et sous atmosphère contenant 5% de CO2. Des cultures pures de mélanocytes sont obtenues au bout de 2 à 3 semaines.

### 2. Temps de contact, irradiation des cellules et préparation des lames

Toutes les compositions testées sont solubilisées dans du DMSO aux concentrations maximales permises. Des essais préliminaires sont effectués pour déterminer les concentrations subtoxiques sur les kératinocytes. La concentration finale en DMSO est toujours inférieure à 2%.

Les mélanocytes sont mis en contact avec le produit pendant 30 min à 37°C, puis ils sont irradiés par des UVA. Les irradiations UVA sont générées par un irradiateur UV Bio-Sun (Vilbert Lourmat, Marne la Vallée). Cet appareil est équipé de lampes monochromatiques qui émettent à une longueur d'onde de 312 nm et/ou 365 nm. Les lampes délivrent une énergie calculée, à l'aide d'un radiomètre de type RMW-365/312. Les énergies délivrées sont de 0.8 J/cm2 pour les UVA. Le test dit « des comètes » est réalisé immédiatement après l'exposition. Deux types de contrôles sont inclus dans ces expériences :
- Contrôles négatifs : mélanocytes non traités mais irradiés par les UVA; mélanocytes traités pendant 30 min avec la composition testée, mais non irradiés.
- Contrôle positif : mélanocytes non traités mais irradiés.

Après un traitement avec un mélange trypsine/EDTA (0,05 % / 0,02 %) pendant 2 à 3 minutes, les cultures sont récupérées par centrifugation et placées dans du tampon PBS sans Ca++ et sans Mg++ (Sigma). Suivant une seconde centrifugation, les cellules (4,5-5,0 x 104 cellules) sont placées en suspension dans 0,5 % agarose Low Melting Point, LMP (Sigma). Le mélange est directement déposé sur des lames de microscope recouvertes d'une pré-couche d'agarose (1,6 %) séchée, pendant une nuit à température ambiante et fraîchement précoatée avec une seconde couche d'agarose (0,8 %).

### 3. Le test des comètes (technique des lames sèches) et traitement enzymatique

Le protocole du test des comètes est celui de De Méo et Coll [1] en incorporant la technique des « lames sèches » [2]. Les lames sont placées après les irradiations dans un bain de lyse (2,5 M NaCl, 100 mM Na2EDTA, 10 mM Tris-HCl pH 10, 1 % de sodium sarcosinate, 1 % de triton X-100 et 10 % de DMSO). La lyse cellulaire s'effectue à 4 °C pendant 60 minutes, suivie par la dénaturation de l'ADN à température ambiante pendant 20 min dans une solution fortement alcaline (1 mM Na2EDTA et 300 mM NaOH, pH > 13.0). Après une électrophorèse (25V, 300 mA) pendant 20 min, les lames sont neutralisées par le tampon Tris-HCl (04 M; pH 7,4) et déshydratées dans de l'éthanol ou du méthanol absolu.

### 4. Observation microscopique et analyse d'image

Les lames sont colorées par une solution de bromure d'éthidium (75 µl de 2 µg/ml) et observées à l'aide d'un microscope à fluorescence BH2-RFL (Olympus, Japon) équipé d'un filtre dichroïque 20BG-W (excitation : 515-560 nm ; émission : 590 nm) et d'un objectif Apo D-Plan 20x. L'analyse d'image s'effectue avec une caméra CCD monochrome haute sensibilité (Cohu 4912-5000) couplée à une carte d'acquisition Matrox IP-8. L'ensemble est piloté par le logiciel Fenestra Komet (Kinetic Imaging, Liverpool, RU, version 3.1).

Un total de 100 cellules par échantillon (50 cellules/lame) est analysé. Le paramètre utilisé est « Tail DNA », qui est défini comme le pourcentage d'ADN dans la queue de la « comète ». Pour chaque série d'expériences, un contrôle négatif (cellules non irradiées) et un contrôle positif (cellules irradiées sans composition testée) sont inclus.

### 5. Analyse statistique

Des régressions non linéaires basées sur une fonction *χ*² sont calculées directement à partir des fréquences de distribution des TM (« tail moment ») pour chaque échantillon. En effet, Bauer et Coll [3] ont récemment montré que ces distributions suivaient une fonction *χ*². Cette méthode est basée sur une analyse de la distribution selon une loi de *χ*².

Le facteur n (aussi appelé *χ*² TM) qui représente le degré de liberté de la fonction est directement corrélé avec le degré de lésion (TM moyen). Le facteur n varie de 2 (cellules intactes) à 15 (cellules extrêmement lésées avec une fréquence de distribution gaussienne).

Le degré de liberté (n) peut être utilisé comme un indicateur de lésion de l'ADN. Les fréquences de distribution sont calculées avec le Tableur Excel 97 (Microsoft) et les régressions non linéaires sont calculées avec le logiciel Table Curve 2D (Jandel Scientific, version 5.0)

### 6. Références

[1] De Méo M, M. Laget M, Castegnaro M, Duménil G. Genotoxic activity of potassium permanganate acidic sodium. Mutation Res. 1991; 260; 295-306.
[2] Klaude M, Ericksson S, Nygren J, Annstrom G. The cornet assay: mechanism and technical considération. Mutation Res. 1996; 363; 89-96.
[3] Bauer E, Recknagel RD, Fiedler U, Wollweber L, Bock C, Greulich KO. The distribution of the tail moments in single cell electrophoresis (cornet assay) obeys a chi-square (χ2) not a gaussian distribution. Mutation Res. 1998; 398: 101-110.

### 7. Degré de protection des compositions testées

**Les résultats selon le tableau ci-dessous sont obtenus.**

| **Composition** | **OTM-***_{χ}*² | **Degré de protection (%)** |
|---|---|---|
| NI | 2.08 ± 0.02 | - |
| NI + ATP (4 mM) | 2.08 ± 0.02 | - |
| NI + Citru (4 mM) | 2.06 ± 0.02 | - |
| NI+ATP (4 mM)+Citru(4 mM) | 2.07 ± 0.02 | - |
| UVA | **9.16 ± 0.32.** | **0.00 %** |
| **UVA** + **ATP** (4 mM) | 6.31 ± 0.38 | **14.6% (NS)** |
| **UVA** + Citru (4 mM) | 2.22 ± 0.20 | 67.7 % |
| UVA+ATP (4 mM)+Citru(4 mM) | 2.11 ± 0.04 | 99.,6 % |

| | | |
|---|---|---|
| OTM *χ*² = Tail Moment *χ*² : Degré de liberté de la fonction calculé par régression non linéaire de la fréquence normalisée de distribution des OTM. La probabilité des modèles dans tous les cas est de P < 0.001. NI : mélanocytes non irradiés NI + ATP : mélanocytes non irradiés et prétraités par de l'ATP (4 mM) pendant 30 min NI + Citru : mélanocytes non irradiés et prétraités par de la Citrullyl-arginine (4 mM) pendant 30 min UVA : mélanocytes irradiés par des UVA (0,8 J/cm2) UVA + ATP : mélanocytes irradiés par des rayonnements UVA (0,8 J/cm2) et prétraités par de l'ATP (4 mM) pendant 30 min UVA + Citru : mélanocytes irradiés par des rayonnements UVA (0,8 J/cm2) et prétraités par de la Citrullyl-arginine (4 mM) pendant 30 min NS : différence non significative entre UVA + Citru et UVA + ATP. Le degré de protection de la Citrullyl-arginine est très supérieur à celui de l'ATP. La synergie entre la molécule d'ATP et le peptide est très nette. | | |

Les exemples qui suivent illustrent l'invention sans en limiter pour autant la portée.

### EXEMPLES

### Exemple 1: Crème anti rides (selon l'invention)

| | |
|---|---|
| Stéarate de sucrose | 0,5-6 % |
| Distéarate de sucrose | 0,5-6 % |
| **ATP disodique** | **0.01-0.05 %** |
| **Diguanosine tétraphosphate (Gp4G)** | **0**.**5 - 1%** |
| Triglycéride caprylique/caprique | 3-15 % |
| Triglycéride caprylique/caprique/succinique | 3-15 % |
| Céramides 3 | 0,05-1 % |
| Palmitate d'ascorbyle | 0,01-0,1 % |
| Acétate de tocophéryle | 0,05-1 % |
| Urée | 0,5-2 % |
| Chlorure de calcium | 0,05-0,5 % |
| Chlorure de magnésium | 0,05-0,5 % |
| ***β-alanyl-I-histidine*** (Carnosine) | 0.5 - 1 % |
| | |
| ***Gly-His-Lys (peptide powder)*** | ***10 - 5 ppm*** |
| Sérine | 0,2-2 % |
| Glycérine | 0,5-3 % |
| Acide citrique | 0,1-0,5 % |
| Citrate trisodique | 0,5-1,5 % |
| Palmitate de vitamine A | 0,1-0,5 % |
| Phospholipides | 0,1-0,4 % |
| Superoxyde dismutase | 0,5-2 % |
| Hyaluronate de sodium | 0,5-3 % |
| Sorbate de potassium | 0,2-0,5 % |
| Gomme sclerotium | 0,1-0,5 % |
| Gomme xanthane | 0,1-0,5 % |
| Eau | qsp.100 % |
| Parfum | qs. |
| Conservateurs | qs. |

### Exemple 2: Lait hydratant (hors invention)

| | |
|---|---|
| **Diguanosine tétraphosphate (Gp4G)** | **03 - 1%** |
| Phospholipides | 3 % |
| Céramide 3 | 0,1 % |
| Palmitate de vitamine A | 0,15 % |
| Stéareth-20 | 0,2 % |
| Stéareth-2 | 1 à 3 % |
| Methyl paraben | 0,25 % |
| Chlorure de calcium | 0,01 % |
| Chlorure de magnésium | 0,01 % |
| Eau | qsp.100 % |
| Alcool cétostéarylique | 2 à 4 °A |
| Myristate de myristyle | 2 à 4 % |
| Myristate d'isopropyle | 4 % |
| Glycérine | 1 % |
| **L-Citrullyl-L-Arginine** | **0,1 à 2 %** |
| Dimethicone | 0,5 % |
| Alcools lanoliniques | 0,5 % |
| Propyl paraben | 0,25 % |

### Exemple 3: Crème hydratante (hors invention)

| | |
|---|---|
| **ATP disodique** | **0.01-0.05 %** |
| Oléate de sorbitane | 3,5 % |
| Polysorbate 80 | 2 à 4 % |
| Huile de germe de blé | 3% |
| Huile d'amandes douces | 5 % |
| Myristate d'isopropyle | 12 % |
| Phospholipides | 0,5 % |
| Céramides 3 | 0,1 % |
| Polyacrylamide & C₁₄₋₁₃ isoparaffine & laureth-7 | 2 à 3,5 % |
| Palmitate de vitamine A | 0,1 % |
| Tocopherol | 0,05 % |
| PCANa | 0,5 % |
| **Diguanosine tétraphosphate (Gp4G)** | **0.5 - 1 %** |
| Hyaluronate de sodium | 0,1 % |
| Eau | qsp.100 % |
| **L-Citrullyl-L-Arginine** | **0,1 à 2 %** |
| Conservateurs | qsp |
| Parfum | qsp |

### Exemple 4 : Crème bronzante solaire protectrice (selon l'invention)

| | |
|---|---|
| **ATP disodique** | **0.01-0.05 %** |
| Méthoxycinnamate d'octyle | 6,00 % |
| (Neo Heliopan AV) | |
| Butyl-méthoxydibenzoylméthane | 3,00 % |
| (Parsol 1789) | |
| Octyl-triazone | 2,00 % |
| (Uvinul T150) | |
| Di-C12-13 Alkyl Tartrate | 8,00 °A |
| (Cosmacol ETI) | |
| Alcool cétylique | 0,50 % |
| Diméthicone | 0,50 % |
| Coco caprylate/caprate | 8,00 % |
| PVP/Copolymère Eicosène | 2,00 % |
| Cétyl-phosphate de potassium | 2,00 % |
| Méthyl- et propyl-paraben | 0,25 % |
| Disodium EDTA | 0,10 % |
| BHT | 0,05 % |
| Carbomer | 10,00 % |
| **Diguanosine tétraphosphate (Gp4G)** | 0.5 - 1% |
| **Alanyl-I-histidine** (Carnosine) | 0.8 - 1% |
| Propylène glycol | 5,00 % |
| Hydroxyde de potassium | 4,05 % |
| Acide phénylbenzimidazole sulfonique | 2,00 % |
| (Eusolex 232) | |
| Acétate de tocophéryle | 2,50 % |
| Panthénol | 1,00 % |
| MSH (Ala-His-Lys-Phe-Tyr) | 0.0001 - 0.00001 % |
| ***Photolyase*** | ***0.1%*** |
| Eau | qsp. 100 % |
| Parfum | qs |

### Exemple 5: Crème solaire photo-protectrice et réparatrice (selon l'invention)

| | |
|---|---|
| **ATP disodique** | 0.01-0.05 % |
| Ethoxy-diglycol et concombre | 8,00 % |
| Di-C12-13 Alkyl Tartrate | |
| (Cosmacol ETI) | 5,00 % |
| Méthoxycinnamate d'octyle | |
| (Parsol MCX) | 5,00 % |
| Butyl-méthoxydibenzoylméthane | |
| (Parsol 1789) | 2,00 % |
| Diméthicone triméthylsiloxysilicate | 3,00 % |
| Acétate de tocophéryle | 0,20 % |
| Distéarate de sucrose | 5,00 % |
| Hexylene Glycol | 5,00 % |
| Butyl-, Méthyl-, Propyl-paraben + Phénoxyéthanol | 0,40 % |
| ***L-Citrullyl-L-Arginine Eau*** | ***0,1 à 2%*** |
| qsp.100 % | |
| **Diguanosine tétraphosphate (Gp4G)** | **1-1.5 %** |
| **MSH (Ala-His-Lys-Phe-Tyr)** | **0.0001** - **0.00001 %** |
| Endonucléase | 0.2% |
| Alanyl-I-histidine (Carnosine) | 0.5-1 % |
| Parfum qs. | |

### Exemple 6 : Lait apaisant photo-réparateur (hors invention)

| | |
|---|---|
| Huile minérale | 2,00 % |
| **Diguanosine tétraphosphate (Gp4G)** | **0.5 - 1%** |
| Di-C12-13 Alkyl Tartrate | 4,00 °A |
| (Cosmacol ETI) | |
| Stéarate d'octyle | 3,00 °A |
| Isoamyl-p-méthoxycinnamate | 5,00 % |
| (Parsol MCX) | |
| Butyl-méthoxydibenzoylméthane | 1,00 % |
| (Parsol 1789) | |
| Diisostéarate de polyglycéryl-3 | 4,00 °A |
| PEG-20 Laurate de glycéryle | 1,00 % |
| Carbomer | 0,4 % |
| Propylène glycol | 2,00 % |
| Conservateurs | 0,50 % |
| Gomme de xanthane | 0,30 % |
| Triéthanolamine | 0,85 % |
| Acide phénylbenzimidazole-sulfonique | 2,5 % |
| (Neo Heliopan Hydro) | |
| Acétyl-tyrosine | 2,00 % |
| **Alanyl-I-histidine (Carnosine)** | **0.5 -1 %** |
| **Gly-His-Lys (peptide powder)** | **10 - 5 ppm** |
| Eau qsp.100% | |
| Parfum | qs. |

### Exemple 7 : Crème visage désensibilisante (hors invention)

| | |
|---|---|
| Triglycéride caprylique/caprique/succinique | 1 à 10 % |
| Palmitate d'ascorbyle | 0,01 à 0,1 % |
| Stéarate de glycéryle | 1 à 5 % |
| Acide stéarique | 1 à 5 % |
| Acétate de tocophérol | 0,1 à 1 % |
| Triglycéride caprylique/caprique | 1 à 15 % |
| **ATP disodique** | **0.01-0.05 %** |
| Pyridoxine | 0,01 à 0,05 % |
| Acide citrique | 0,1 à 0,5 % |
| Gluconate de zinc | 0,1 à 1 % |
| Citrate trisodique | 1 à 2,5 % |
| | |
| *L-Citrullyl-L-Arginine* | ***0,1 à 2* %** |
| **Diguanosine tétraphosphate (Gp4G)** | **0.5 - 1%** |
| Glycérine | 1 à 4 % |
| Palmitate de vitamine A | 0,01 à 1 % |
| d-Panthénol | 0,1 à 1 % |
| Rhamnose | 0,1 à 1 % |
| L-Fucose | 0,01 à 1 % |
| Lactoferrine / Lactoperoxydase | 0,01 à 1 % |
| Superoxyde dismutase | 0,01 à 1 % |
| Polyacrylamide / c13-14 isoparaffine / Laureth-7 | 0,2 à 1 % |
| Eau | qsp.100 % |

### Exemple 8 : Lait corporel apaisant (selon l'invention)

| | |
|---|---|
| Polymère d'acide acrylique | 0,1 - 1,5 % |
| Acide glycyrrhétinique • | 0,1 - 1 % |
| Triéthanolamine | 0,1 - 2 % |
| Butylène glycol | 0,5 - 4 % |
| **ATP disodique** | **0.01-0.05 %** |
| Palmitate d'ascorbyle | 0,01 à 0,1 % |
| Acétate de tocophérol | 0,1 à 1 % |
| Pyridoxine | 0,01 à 0,05 % |
| Acide citrique | 0,1 à 0,5 % |
| Gluconate de zinc | 0,1 à 1 % |
| Citrate trisodique | 1 à 2,5 % |
| L-Arginine | 0,1 à 2 % |
| Palmitate de vitamine A | 0,01 à 1 % |
| d-Panthénol | 0,1 à 1 % |
| L-Fucose | 0,01 à 1 % |
| Lactoferrine / Lactoperoxydase | 0,01 à 1 % |
| ***Alanyl-I-histidine*** (Carnosine) | 0.5 - 1 % |
| | |
| ***R-Gly-Gln-Pro-Arg*** | ***15* - *20 ppm*** |
| Superoxyde dismutase | 0,01 à 1 % |
| Sorbate de potassium | 0,1 à 0,6 % |
| Conservateurs | qs. |
| Eau | qsp.100 % |

### Exemple 9 : Crème apaisante peau grasse (hors invention)

| | |
|---|---|
| Propylène glycol | 1 - 8 % |
| Monolaurate de sorbitan | 0,5 - 5 % |
| Diméthicone copolyol | 0,1 - 5 % |
| Acide salicylique | 0,1 - 0,5 % |
| Disodium EDTA | 0,05 - 0,5 % |
| **Diguanosine tétraphosphate (Gp4G)** | 0.5 -1 % |
| **ATP disodique** | **0.01-0.05 %** |
| Gluconate de zinc | 0,1 - 1 % |
| Palmitate d'ascorbyle | 0,01 à 0,1 % |
| Acétate de tocophérol | 0,1 à 1 % |
| Pyridoxine | 0,01 à 0,05 % |
| Acide citrique | 0,1 - 0,5 % |
| Chlorure de sodium | 0,1 - 1,5 % |
| Citrate trisodique | 1 à 2,5 % |
| L-Arginine | 0,1 à 2 % |
| Palmitate de vitamine A | 0,01 à 1 % |
| d-Panthénol | 0,1 à 1 % |
| Rhamnose | 0,1 à 1 % |
| L-Fucose | 0,01 à 1 % |
| Lactoferrine I Lactoperoxydase | 0,01 à 1 % |
| | |
| ***Gly-His-Lys (peptide powder)*** | ***10 - 5 ppm*** |
| Superoxyde dismutase | 0,01 à 1% |
| Conservateurs | qs. |
| Eau | qsp.100 % |

### Exemple 10 : Lotion démaquillante (selon l'invention)

| | |
|---|---|
| **Diguanosine tétraphosphate (Gp4G)** | **1 - 2%** |
| | |
| ***ATP* disodique** | ***0.1-0.5 %*** |
| Citrate trisodique | 1 à 2,5 % |
| Glycérine | 0,5-3 % |
| Hexylene glycol | 4-5% |
| d-Panthénol | 0,1 à 1 % |
| ***Alanyl-I-histidine*** (Carnosine) | 0.5 - 1 % |
| Conservateurs (Methyl parabenne et phenoxyethanol) | qs. |
| Eau | qsp.100 % |

## Revendications

1. Composition cosmétique comprenant un système bioactif associant :
- d'une part une forme stable en solution aqueuse d'ATP (adénosine triphosphate) sous la forme d'un sel de sodium d'ATP, en présence éventuellement de Gp₄G (diguanosine tétraphosphate) ou d'Ap₄A (diadénosine tétraphosphate) ; et
- d'autre part au moins un dipeptide biomimétique, mimant soit un polypeptide cutané ou une protéine cutanée, soit une biomolécule agoniste ou antagoniste audit polypeptide cutané ou à ladite protéine cutanée, ledit dipeptide biomimétique étant la β-alanyl-L-histidine.

2. Composition selon la revendication 1, ***caractérisée* en ce que** le sel de sodium d'ATP est un sel disodique.

## Patentansprüche

1. Kosmetische Zusammensetzung, die ein bioaktives System umfasst, das miteinander verbindet:
- erstens eine stabile Form in wässriger Lösung von ATP (Adenosintriphosphat) in Form eines ATP- Natriumsalzes, eventuell in Anwesenheit von Gp**4**G (Diguanosintetraphosphate) oder Ap**4**A (Diadensosintetraphosphate); und
- zweitens mindestens ein biomimetisches Dipeptid, das entweder ein dermales Polypeptid oder ein dermales Protein, oder ein Agonisten- oder Antagonisten-Biomolekül dieses dermalen Polypeptid oder dermalen Proteins nachahmt, wobei es sich bei diesem biomimetischen Dipeptid um β-alanyl-L-histidin handelt.

2. Zusammensetzung nach Anspruch 1, ***dadurch gekennzeichnet, dass*** es sich bei dem ATP-Natriumsalz um ein Dinatriumsalz handelt.

## Claims

1. A cosmetic composition comprising a bioactive system that combines:
- firstly, a stable form, in aqueous solution, of ATP (adenosine triphosphate) in the form of a sodium salt of ATP with, optionally, Gp₄G (diguanosine tetraphosphate) or Ap₄A (diadenosine tetraphosphate); and,
- secondly, at least one biomimetic dipeptide that mimics a cutaneous polypeptide or a cutaneous protein, or a biomolecule that is an agonist or antagonist with respect to said cutaneous polypeptide or to said cutaneous protein, wherein the biomimetic dipeptide is β-alanyl-L-histidine.

2. The composition as claimed in claim 1, **characterized in that** the sodium salt of ATP is a disodium salt.
